# EUROPEAN PATENT APPLICATION

(11) **EP 4 821 911 A1**
(43) Date of publication of application: **14.10.2026**
(21) Application number: 24902554.5
(22) Date of filing: 26.11.2024
(51) Int. Cl.: C07C 209/62, C07C 209/60, C07C 211/55, C07D 215/00

(54) **METHOD FOR CO-PRODUCING DIPHENYLAMINE AND QUINOLINE**

(30) Priority: 13.12.2023 CN 202311712982
(71) Applicant: China Petroleum & Chemical Corporation, Beijing 100728 (CN); Sinopec Dalian Research Institute of Petroleum and Petrochemicals Co., Ltd., Lushunkou District Dalian, Liaoning 116045 (CN)
(72) Inventor: ZHAO, Xiangyu, Dalian, Liaoning 116045 (CN); QI, Wenbo, Dalian, Liaoning 116045 (CN); AI, Fubin, Dalian, Liaoning 116045 (CN); LI, Haomeng, Dalian, Liaoning 116045 (CN); LI, Lanpeng, Dalian, Liaoning 116045 (CN)
(74) Representative: karo IP
(86) International application number: PCT/CN2024/134568
(87) International publication number: WO 2025/124132

(57) **Abstract**

The present invention belongs to the technical field of fine chemical engineering, and particularly relates to a method for co-producing diphenylamine and quinolone. The method comprises: subjecting aniline and a catalyst to a contact reaction in the presence of propylene, so as to obtain a reaction product containing diphenylamine and quinoline. In the method provided in the present invention, by introducing propylene into a reaction system, diphenylamine and quinoline can be co-produced, the conversion rate of aniline is high, and the total selectivity of the diphenylamine and quinoline is high.

## Description

### CROSS-REFERENCE TO RELATED APPLICATIONS

The application claims the benefit of China patent application No. "202311712982.2", filed on December 13, 2023, the content of which is specifically and entirely incorporated herein by reference.

### TECHNICAL FIELD

The present invention belongs to the technical field of fine chemical engineering, and particularly relates to a method for co-producing diphenylamine and quinoline.

### BACKGROUND ART

Diphenylamine is a widely used chemical product, and has important applications in rubber additives, dyes, explosive stabilizers, fruit preservatives, medicaments and other fields. Up to now, there are more than 20 methods for synthesizing diphenylamine, and the raw materials for production comprise aniline, phenol, cyclohexanone, N-cyclohexylaniline, dicyclohexyl and the like, and the catalysts are mainly aluminum chloride, boron trifluoride, activated alumina, zeolite molecular sieves, etc. Among these methods, the method of producing diphenylamine with single aniline as the raw material has the best atomic economy and the strongest feasibility for large-scale production, and has been industrialized. The production process of synthesizing diphenylamine from aniline is divided into intermittent method, gas phase continuous method, and liquid phase continuous method, wherein the liquid phase continuous method is the most advanced production process at present. However, the aniline conversion rate in the production process is low, which seriously limits the production efficiency of the process of synthesizing diphenylamine from aniline, it is also the main problem encountered in the current industrial production process of diphenylamine.

CN1186802A discloses a process for continuous synthesis of diphenylamine from aniline in the presence of hydrogen, wherein the process uses the raw material refined gas (containing about 75% hydrogen gas and 25% nitrogen gas) of the synthetic ammonia plant as the hydrogen gas source, and forms a gas phase circulation system between the diphenylamine device and the synthetic ammonia compression section. The advantage of the process is that it effectively improves the aniline conversion rate in the diphenylamine production process and prolongs the service life of said catalyst on the basis of making full use of hydrogen gas resources. Although the hydrogenation effect is relatively obvious, the aniline conversion rate still cannot exceed 30%.

CN103044270A also discloses a method for continuously synthesizing diphenylamine by using phenylamine in the presence of hydrogen, and the purpose of improving the conversion rate of phenylamine is achieved by developing a hydrogen dissolving process. The key point of said process is contacting the phenylamine raw material with hydrogen gas before entering into a reactor, dissolving the hydrogen gas into the phenylamine raw material, and the phenylamine raw material with the dissolved hydrogen gas enters into a reactor used for continuously synthesizing diphenylamine from phenylamine, and carries out the reaction of synthesizing diphenylamine with phenylamine. The advantage of this method is that the efficiency of synthesizing diphenylamine from phenylamine is improved by dissolving hydrogen. However, the conversion rate of phenylamine cannot be effectively improved by dissolving hydrogen.

Quinoline is an important aromatic heterocyclic compound. Quinoline compounds based on the quinoline structure have been widely used and studied in many fields such as coordination chemistry, metal organic chemistry, asymmetric organic synthesis chemistry, and material chemistry. In actual production and daily life, the quinoline compounds have played an important role in many fields such as medicament, biological reagents, and dyes. In industrial production, quinoline is mainly prepared through coal tar extraction, Skraup synthesis, Doebner-Von Miller synthesis, and nitrobenzene reductive carbonylation. Although quinoline and quinoline compounds are widely used, the production process has problems such as high energy consumption and heavy pollution.

The literature (Tetrahedron Letters, 2014, 5(22), 3319-3321) discloses a method for synthesizing quinoline. The method adopts acrolein diethyl acetal and aniline as raw materials, uses metal-loaded zeolite as a solid catalyst, and synthesizes quinoline compounds with microwave assistance, and the product yield reaches 83.1%. Although the method can obtain quinoline products with a high yield, the microwave-assisted synthesis conditions are not yet suitable for industrial production.

### SUMMARY OF THE INVENTION

The present invention aims to solve the problems in the prior art with respect to a low aniline conversion rate and a low added value of product, and provides a method for co-producing diphenylamine and quinoline. The method has the characteristics of high conversion rate of aniline, high total selectivity of diphenylamine and quinoline, high added value of product, and rosy industrial application prospects.

The present invention provides a method for co-producing diphenylamine and quinoline, the method comprises: subjecting aniline and a catalyst to a contact reaction in the presence of propylene, so as to obtain a reaction product containing diphenylamine and quinoline.

Both diphenylamine and quinoline can be produced using aniline as a raw material. Among the currently mature chemical production processes and technologies, only the process of continuously synthesizing diphenylamine from aniline can simultaneously generate diphenylamine and quinoline, but the yield of quinoline is extremely low, usually below 1%, and cannot meet actual needs. The method for co-producing diphenylamine and quinoline provided by the present invention can effectively improve the conversion rate of aniline and the selectivity of quinoline by introducing propylene into the reaction system, the total selectivity of diphenylamine and quinoline is high, and the added value of the product can be greatly improved.

### DESCRIPTION OF THE PREFERRED EMBODIMENT

The terminals and any value of the ranges disclosed herein are not limited to the precise ranges or values, such ranges or values shall be comprehended as comprising the values adjacent to the ranges or values. As for numerical ranges, the endpoint values of the various ranges, the endpoint values and the individual point values of the various ranges, and the individual point values may be combined with one another to produce one or more new numerical ranges, which should be deemed to have been specifically disclosed herein.

The present invention provides a method for co-producing diphenylamine and quinoline, the method comprises: subjecting aniline and a catalyst to a contact reaction in the presence of propylene, so as to obtain a reaction product containing diphenylamine and quinoline.

Both diphenylamine and quinoline can be produced using aniline as a raw material. However, among the currently mature chemical production processes and technologies, the yield of quinoline in the process of continuously synthesizing diphenylamine from aniline is extremely small and difficult to separate. For example, CN103044270A discloses a process of synthesizing diphenylamine from phenylamine, wherein the conversion rate of phenylamine is less than 30%, and the selectivity of quinoline in the reaction solution is less than 1%. Therefore, a very small amount of by-product quinoline can hardly be recovered and utilized as a high-value product.

The inventors of the present invention have found in research that, subjecting aniline and a catalyst to a contact in the presence of propylene, carrying out the aniline condensation reaction to obtain diphenylamine, aniline and propylene can perform reaction to generate quinoline, thereby achieving the co-production of diphenylamine and quinoline, and obtaining more high value-added products while improving the conversion rate of the raw material aniline. Therefore, the method provided by the present invention can be carried out in the original aniline condensation device, both the conversion rate of aniline and the total selectivity of diphenylamine and quinoline are high. In addition, the method provided in the present invention does not produce by-products such as water during the reaction process, thus avoiding poisoning of the catalyst and improving stability of the reaction.

According to some preferred embodiments of the present invention, the molar ratio of propylene to aniline is 1: (1-5), such as 1:1, 1:1.5, 1:2, 1:2.5, 1:3, 1:3.5, 1:4, 1:4.5, 1:5 and other specific but non-limiting ratios. Preferably, the molar ratio of propylene to aniline is 1:(1.5-3). The above preferred embodiments are beneficial to further improve the conversion rate of aniline, improve the total selectivity of diphenylamine and quinoline, and reduce the occurrence of side reactions.

The present invention has a wide selection range for the contact reaction conditions. Preferably, the contact reaction temperature is within the range of 190-320°C, more preferably within the range of 230-280°C, the liquid hourly volume space velocity of aniline is within the range of 50-180h⁻¹, more preferably within the range of 80-150h⁻¹, and the reaction pressure is within the range of 3-8MPa, more preferably within the range of 5-7 MPa. Under the above preferred conditions, the conversion rate of raw material aniline can reach 35mol% or more.

The diphenylamine and quinoline in the product obtained with the method provided by the invention can be separated by any conventional method in the art (e.g., by distillation), which is not particularly limited in the present invention.

According to the present invention, the contact reaction can be carried out in any conventional reactor in the art. Preferably, the contact reaction is performed in a fixed bed reactor, which is filled with a catalyst.

The present invention does not particularly limit the feeding modes of propylene and aniline, those skilled in the art can adjust the feeding modes according to the actual condition and specific requirement of the reactor. For example, both propylene and aniline can be fed into the fixed bed reactor by means of top feed and pass through the catalyst bed; or both propylene and aniline can be fed into the fixed bed reactor by means of bottom feed and pass through the catalyst bed; or propylene may be fed into the fixed bed reactor by means of bottom feed, and aniline may be fed into the fixed bed reactor by means of top feed and passes through the catalyst bed.

In order to further improve the conversion rate of aniline, preferably, both propylene and aniline are fed into the fixed bed reactor by means of bottom feed and pass through the catalyst bed.

The present invention has no particular limitation on the type and composition of the catalyst, any conventional catalyst in the art that can be used for the condensation of aniline to prepare diphenylamine may be applied in the present invention, preferably a solid acid catalyst for the aniline condensation reaction. The present invention has no particular limitation on the source of catalyst, and the catalyst may be commercially available or prepared with any known method in the art.

According to some preferred embodiments of the present invention, the catalyst comprises β zeolite and a metal component.

According to the present invention, preferably, the molar ratio of silica to alumina in said β zeolite is within the range of 20-100, more preferably within the range of 50-70.

Preferably, the β zeolite is Hβ zeolite.

The present invention has a wide selection range for the metal component. In order to further improve the conversion rate of aniline and the total molar selectivity of diphenylamine and/or quinoline, preferably, the metal component is at least one selected from alkali metals, further preferably at least one elected from the group consisting of lithium, sodium and potassium.

According to some preferred embodiments of the present invention, based on the total mass of the catalyst, the content of said β zeolite is within the range of 50-90wt%, such as 50wt%, 55wt%, 60wt%, 65wt%, 70wt%, 75wt%, 80wt%, 85wt%, 90wt% or the specific but not limiting content, or a random value within the range consisting of any two numerical values, and the content of said metal component calculated in terms of element is within the range of 0.1-40wt%, for example, it may be 0.1wt%, 0.5wt%, 1wt%, 2wt%, 3wt%, 5wt%, 10wt%, 15wt%, 20wt%, 25wt%, 30wt%, 35wt%, 40wt% or the specific but not limiting content, or a random value within the range consisting of any two numerical values.

Further preferably, based on the total mass of said catalyst, the content of said β zeolite is within the range of 65-75wt%, and the content of said metal component calculated in terms of element is within the range of 1-30wt%. Under the preferable condition, it is beneficial to ensure that the conversion rate of the raw material aniline is higher than 30%, and under certain process conditions, the side reactions are suppressed to obtain more high value-added products.

In the invention, the content of metal components in the catalyst is measured by an inductively coupled plasma optical emission spectrometer (ICP-OES) method.

According to some preferred embodiments of the present invention, the catalyst further comprises a binder to obtain a catalyst molding. The present invention has a wide selection range for the binder, and any binder that can be used for catalyst molding in the art may be applied in the present invention. Preferably, the binder is alumina and/or silica, more preferably alumina.

Preferably, the content of said β zeolite is within the range of 50-90wt%, more preferably within the range of 65-75wt%, the content of said alkali metal component calculated in terms of metal element is within the range of 0.1-40wt%, more preferably within the range of 1-30wt%, and the content of said binder is within the range of 0.5-49.9wt%, more preferably within the range of 5-30wt%, based on the total mass of said catalyst. Under the above preferred composition, it is beneficial to ensure that the conversion rate of the raw material aniline is higher than 30%, and under certain process conditions, the side reaction is suppressed to obtain more high value-added products.

The present invention has no particular limitation on the shape of the catalyst, those skilled in the art can select the size and morphology of the catalyst according to actual needs.

Preferably, the catalyst may be strip-shaped or spherical particles. When the catalyst is strip-shaped, the cross-section of said catalyst may be cylindrical, clover-shaped or four-leaf clover-shaped. The cross-sectional width of the strip-shaped particles is within the range of 0.5-3.0mm, preferably within the range of 1.0-2.0mm. When the catalyst has a spherical shape, the particle diameter is within the range of 0.5-5.0mm, preferably within the range of 1.0-3.0mm.

The invention has no particular limitation on the source of the catalyst, which is commercially available or prepared with any known method in the art. For example, the catalyst can be prepared with the method disclosed in CN200510047489.9. For example, the catalyst of the present invention can be prepared with the following method:
(1) Contacting the Hβ zeolite with a solution of a soluble compound of the metal component;
(2) Subjecting the mixture obtained in step (1) to solid-liquid separation, washing and drying;
(3) Fully kneading and molding and then drying and calcining the modified Hβ zeolite obtained in step (2), a binder and/or a binder precursor and a processing aid (e.g., an extrusion aid and a peptizing agent).

Preferably, the contact temperature of step (1) is within the range of 15-35°C, and the time is within the range of 0.5-2h. For example, the contact may be performed at room temperature.

Preferably, the concentration of the solution of the soluble compound of the metal component is within the range of 0.05-4mol/L.

The present invention has no particular limitation on the operation mode and conditions of the solid-liquid separation and washing, those skilled in the art can select them according to actual needs. The solid-liquid separation can be performed by filtering, for example. The present invention has no particular limitation on the number of washing times, and it can be adjusted according to the actual washing situation. The detergent used for washing may be water.

The present invention has no particular limitation on the drying conditions in step (2). Preferably, the drying temperature is within the range of 100-130°C and the drying time is within the range of 8-12h.

In the invention, the binder precursor refers to any substance that can be calcined to obtain the binder. For example, the precursor of alumina may be pseudo-boehmite and/or aluminum hydroxide, which are well known among those skilled in the art.

The invention has no particular limitation on the processing aid, which may be a conventional choice in the art. The invention has no particular limitation on the dosage of said processing aid, those skilled in the art can select the dosage according to actual needs. The extrusion aid, for example, may be sesbania powder, and the peptizing agent may be dilute nitric acid and/or citric acid, wherein the dilute nitric acid generally has a concentration within the range of 3-15 wt%.

The present invention has no particular limitation on the molding mode, those skilled in the art can select the molding mode according to actual needs, for example, the molding may be extrusion molding, tablet molding, ball rolling molding.

Preferably, the drying temperature in step (3) is within the range of 100-130°C and the drying time is within the range of 8-12h.

Preferably, the calcination temperature in step (3) is within the range of 450-550°C, and the calcination time is within the range of 6-8h.

On the other hand, the present invention provides a method for co-producing diphenylamine and quinoline from aniline, the method comprises the following steps: subjecting the raw materials aniline and propylene to passing through a fixed bed reactor containing a Hβ zeolite catalyst under the reaction conditions comprising a reaction temperature within the range of 190°C-320°C and an aniline liquid hourly volume space velocity within the range of 50h⁻¹ -180h⁻¹.

The present invention will be described in detail below with reference to examples.

Unless otherwise specified in the present invention, the raw materials used in the following examples and comparative examples were commercially available.

In the following examples and comparative examples, the calculation methods of the conversion rate of aniline, the selectivity of diphenylamine, and the selectivity of quinoline were as follows:

The molar conversion rate of aniline:
$ω = \frac{{n}_{0} - {n}_{2}}{{n}_{0}} \times 100 %$

Wherein *ω* denoted the molar conversion rate of aniline, mol%; n₀ denoted the mole number of aniline in the raw materials; n₂ denoted the mole number of aniline in the reaction solution.

The molar selectivity of diphenylamine:
${ε}_{1} = \frac{{n}_{3}}{{n}_{1}} \times 100 %$

Wherein *ε*₁ denoted the molar selectivity of diphenylamine, mol%; n₁ denoted the mole number of each product component in the reaction solution, mol; n₃ denoted the mole number of diphenylamine in the reaction solution, mol.

The molar selectivity of quinoline:
${ε}_{2} = \frac{{n}_{4}}{{n}_{1}} \times 100 %$

Wherein *ε*₂ denoted the molar selectivity of quinoline, mol%; n₁ denoted the mole number of each product component in the reaction solution, mol; n₄ denoted the mole number of quinoline in the reaction liquid, mol.

The reaction liquid referred to the mixed liquid phase obtained after the reaction. The "product" in the above formulae did not include aniline. The composition of the reaction liquid was analyzed with reference to the Chemical Industry Standard HG/T5534-2019 in China.

The catalysts used in the examples and comparative examples were prepared with the following method:
(1) Contacting Hβ zeolite with a sodium chloride solution;
(2) Filtering, washing and drying the mixture obtained in step (1);
(3) Fully kneading and extrusion molding the modified Hβ zeolite obtained in step (2), the alumina binder precursor, the extrusion aid and the peptizing agent to obtain a strip-shaped catalyst with a diameter of 2mm and a length of 2-3mm, and then drying and calcining the strip-shaped catalyst.

Wherein the contact temperature of step (1) was 20°C and the contact time was 1.5 hours. The concentration of the sodium chloride solution was 0.5mol/L. Step (2) related to drying at 120°C for 10 hours, and step (3) involved with drying at 120°C for 10 hours and calcining at 500°C for 6 hours. The binder in use was aluminum hydroxide, and the zeolite in use was commercially available Hβ zeolite, which had a specific surface area of 650m²/g and a particle diameter of 1.5-2mm.

The properties of the catalyst were listed in Table 1.

**Table 1**

| Catalysts | | A | B | C | D |
|---|---|---|---|---|---|
| Metal Components | Types | Na | Na | Na | Na |
| | Content (wt%) | 2 | 5 | 20 | 0.5 |
| Hβ zeolite (wt%) | | 72 | 72 | 65 | 65 |
| Molar ratio of silica to alumina in Hβ zeolite | | 50 | 50 | 60 | 60 |
| Alumina | | Balance | Balance | Balance | Balance |

### Example 1

In a stainless steel fixed bed reactor with an inner diameter of 20mm and a length of 1000mm, the filling amount of catalyst was 100mL. The reaction raw materials aniline and propylene (commercially available, with a purity larger than 99%) were introduced into the reactor by means of bottom feed. After the reaction product came out from the top of the reactor, it was cooled and then entered the separator. The reaction was carried out for 48 hours under the reaction conditions in Table 2. The composition of the reaction product was analyzed by gas chromatography, and the measured product distribution was shown in Table 2 below.

### Examples 2-7

The method for co-producing diphenylamine and quinoline was performed according to the method in Example 1, except for that the feed modes and the reaction conditions were as shown in Table 2 respectively, after the reaction was carried out for 48 hours, the composition of the reaction product was analyzed by gas chromatography, and the measured product distribution was shown in Table 2 below.

### Examples 8-11

The method for co-producing diphenylamine and quinoline was performed according to the method in Example 1, except for that the feed modes and the reaction conditions were as shown in Table 3 respectively, after the reaction was carried out for 48 hours, the composition of the reaction product was analyzed by gas chromatography, and the measured product distribution was shown in Table 3 below.

### Comparative Example 1

The method for co-producing diphenylamine and quinoline was performed according to the method in Example 1, except for that propylene was replaced with an equimolar amount of hydrogen gas. The specific reaction conditions and results were shown in Table 3.

### Comparative Example 2

Diphenylamine was synthesized according to the method of CN106631829A, wherein the molar ratio of isopropanol to aniline was 1: 1. The specific reaction conditions and results were shown in Table 3.

**Table 2**

| | Examples | | | | | | |
|---|---|---|---|---|---|---|---|
| | 1 | 2 | 3 | 4 | 5 | 6 | 7 |
| Catalysts | A | B | C | B | D | B | B |
| Molar ratio of propylene: aniline | 1:3 | 1:2.5 | 1:1.5 | 1:2 | 1:2 | 1:0.5 | 1:4 |
| Feeding mode | Bottom feed | Bottom feed | Bottom feed | Bottom feed | Bottom feed | Bottom feed | Bottom feed |
| Reaction temperature, °C | 230 | 230 | 280 | 280 | 280 | 280 | 280 |
| Reaction pressure, MPa | 5.0 | 5.5 | 6.5 | 7.0 | 7.0 | 7.0 | 7.0 |
| Reaction time, h | 48 | 48 | 48 | 48 | 48 | 48 | 48 |
| Aniline liquid hourly volume space velocity, h⁻¹ | 80 | 100 | 85 | 100 | 100 | 100 | 100 |
| Aniline conversion rate (mol%) | 36.5 | 37.1 | 38.2 | 37.4 | 35.9 | 36.8 | 35.8 |
| Diphenylamine selectivity (mol%) | 46.8 | 47.2 | 50.2 | 48.3 | 46.0 | 42.6 | 46.2 |
| Quinoline selectivity (mol%) | 45.5 | 46.6 | 45.8 | 46.3 | 45.2 | 46.1 | 45.2 |
| Total selectivity of diphenylamine and quinoline (mol%) | 92.3 | 93.8 | 96 | 94.6 | 91.2 | 88.7 | 91.4 |

**Table 3**

| | Examples | | | | Comparative Examples | |
|---|---|---|---|---|---|---|
| | 8 | 9 | 10 | 11 | 1 | 2 |
| Catalysts | B | A | C | C | A | A |
| Molar ratio of propylene: aniline | 1:2 | 1:3 | 1:1.5 | 1:1.5 | / | / |
| Feeding mode | Top feed | Bottom feed | Bottom feed | Bottom feed | Bottom feed | Bottom feed |
| Reaction temperature, °C | 280 | 200 | 310 | 280 | 230 | 230 |
| Reaction pressure, MPa | 7.0 | 5.0 | 6.5 | 6.5 | 5.0 | 5.0 |
| Reaction time, h | 48 | 48 | 48 | 48 | 48 | 48 |
| Aniline liquid hourly volume space velocity, h⁻¹ | 100 | 80 | 85 | 160 | 80 | 80 |
| Aniline conversion rate (mol%) | 35.6 | 35.3 | 38.8 | 35.5 | 20.2 | 16.5 |
| Diphenylamine selectivity (mol%) | 43.8 | 46.5 | 45.6 | 48.2 | 96.5 | 34.3 |
| Quinoline selectivity (mol%) | 45.8 | 44.8 | 45.5 | 42.8 | 0.07 | <0.1 |
| Total selectivity of diphenylamine and quinoline (mol%) | 89.6 | 91.3 | 91.1 | 91 | 96.57 | ~34.3 |

As can be seen from the results of the aforementioned examples and comparative examples, in the method provided in the present invention, diphenylamine and quinoline can be co-produced by introducing propylene into a reaction system, the conversion rate of aniline is high, and the total selectivity of the diphenylamine and quinoline is high.

The above content describes in detail the preferred embodiments of the present invention, but the present invention is not limited thereto. A variety of simple modifications can be made in regard to the technical solutions of the present invention within the scope of the technical concept of the present invention, including a combination of individual technical features in any other suitable manner, such simple modifications and combinations thereof shall also be regarded as the content disclosed by the present invention, each of them falls into the protection scope of the present invention.

## Claims

1. A method for co-producing diphenylamine and quinoline, is **characterized in that** the method comprises: subjecting aniline and a catalyst to a contact reaction in the presence of propylene, so as to obtain a reaction product containing diphenylamine and quinoline.

2. The method according to claim 1, wherein the molar ratio of propylene to aniline is 1: (1-5), preferably 1: (1.5-3).

3. The method according to claim 1 or 2, wherein the contact reaction temperature is within the range of 190-320°C, preferably within the range of 230-280°C, the liquid hourly volume space velocity of aniline is within the range of 50-180h⁻¹, preferably within the range of 80-150h⁻¹, and the reaction pressure is within the range of 3-8MPa, preferably within the range of 5-7 MPa.

4. The method according to any one of claims 1-3, wherein the contact reaction is performed in a fixed bed reactor.

5. The method according to claim 4, wherein both propylene and aniline are fed into the fixed bed reactor by means of top feed and pass through the catalyst bed; or
both propylene and aniline are fed into the fixed bed reactor by means of bottom feed and pass through the catalyst bed; or
propylene is fed into the fixed bed reactor by means of bottom feed, and aniline is fed into the fixed bed reactor by means of top feed and passes through the catalyst bed.

6. The method according to any one of claims 1-5, wherein the catalyst is an aniline condensation reaction catalyst;
preferably, the catalyst comprises β zeolite and a metal component.

7. The method according to claim 6, wherein the molar ratio of silica to alumina in said β zeolite is within the range of 20-100;
preferably, the β zeolite is Hβ zeolite.

8. The method according to claim 6 or 7, wherein the metal component is at least one selected from alkali metals, preferably at least one elected from the group consisting of lithium, sodium and potassium.

9. The method according to any one of claims 6-8, wherein the content of said β zeolite is within the range of 50-90wt%, and the content of said metal component calculated in terms of element is within the range of 0.1-40wt%, based on the total mass of the catalyst;
preferably, the content of said β zeolite is within the range of 65-75wt%, and the content of said metal component calculated in terms of element is within the range of 1-30wt%, based on the total mass of said catalyst.

10. The method according to any one of claims 7-9, wherein the catalyst further comprises a binder, preferably the binder is alumina and/or silica, more preferably alumina;
preferably, the content of said β zeolite is within the range of 50-90wt%, more preferably within the range of 65-75wt%, the content of said alkali metal component calculated in terms of metal element is within the range of 0.1-40wt%, more preferably within the range of 1-30wt%, and the content of said binder is within the range of 0.5-49.9wt%, more preferably within the range of 5-30wt%, based on the total mass of said catalyst.
